# EUROPEAN PATENT APPLICATION

(11) **EP 4 530 636 A1**
(43) Date of publication of application: **02.04.2025**
(21) Application number: 23461653.0
(22) Date of filing: 28.09.2023
(51) Int. Cl.: G01N 33/94

(54) **A METHOD AND SYSTEM FOR MONITORING A PANEL OF ANTIARRHYTHMIC DRUGS IN BLOOD**

(71) Applicant: Labexperts SP. Z O.O., 80-126 Gdansk (PL); Gdanski Uniwersytet Medyczny, 80-210 Gdansk (PL)
(72) Inventor: Szewczyk, Rafal, 80-126 Gdansk (PL); Lenartowicz, Anna, 80-126 Gdansk (PL); Mironenka, Julia, 80-126 Gdansk (PL); Sobon, Adrian, 80-126 Gdansk (PL); Krupczynska-Stopa, Katarzyna, 80-126 Gdansk (PL); Stopa, Maciej, 80-126 Gdansk (PL); Borkowski, Tomasz, 80-120 Gdansk (PL); Kalinowski, Leszek, 80-120 Gdansk (PL); Marciniak, Ewelina, 80-120 Gdansk (PL); Ploska, Agata, 80-120 Gdansk (PL); Radulska, Adrianna, 80-120 Gdansk (PL)
(74) Representative: Kancelaria Eupatent.pl Sp. z.o.o

(57) **Abstract**

A method for monitoring a panel of antiarrhythmic drugs in a blood of a subject, the method comprising: obtaining a blood sample from the subject; and determining in the blood sample, using liquid chromatography tandem mass spectrometry (LC-MS/MS), a level of at least one antiarrhythmic drug or antiarrhythmic drug metabolite selected from a group consisting of: Amiodarone, Propafenone, Sotalol, Digoxin, Ramipril, Eplerenone, Desetyl-Amiodarone, Hydroxy-Propafenone, Spironolactone, Metoprolol, Perindopril, Carvedilol, Bisoprolol and Nebivolol.

## Description

### TECHNICAL FIELD

The present invention relates to methods and systems for therapeutic drug monitoring, in particular for monitoring a panel of antiarrhythmic drugs in blood.

### BACKGROUND OF THE INVENTION

Therapeutic drug monitoring (TDM) is an interdisciplinary field of medicine that intersects medicinal chemistry and clinical pharmacology. It specializes in measuring drug levels in the blood, with a primary focus on drugs with a narrow therapeutic range, i.e., drugs that can easily be under- or overdosed. TDM aims to improve patient care by individually adjusting the dosage of drugs that clinical experience or trials have shown to improve outcomes in general or special populations. TDM can be based on a priori pharmacogenetic, demographic, and clinical information, and/or on the a posteriori measurement of blood concentrations of drugs (pharmacokinetic monitoring) or biological surrogate or endpoint markers of effect (pharmacodynamic monitoring).

In the case of anti-arrhythmic treatment, current drug dosage regimens consider the patient's body weight and often kidney and liver functions, but they overlook the patient's metabolism. Patients with arrhythmias often suffer from other diseases, including those that may affect the pharmacokinetics of drugs, such as diabetes, liver diseases, or digestive system diseases. The patient's metabolism is crucial for anti-arrhythmic drugs with a narrow therapeutic index. An insufficient dose will result in a lack of therapeutic effect, potentially leading to life-threatening conditions. Conversely, an excessive drug dosage may result in accumulation and lead to the deterioration of liver, renal functions or rapid cardiac death. The importance of TDM increases with the rising number of cardiac arrhythmia cases, estimated at approximately 0.8 million people in Poland and about 13 million people in Europe.

WO2009146450A2 discloses a method for monitoring an immunosuppressant drug level and renal function, hepatic function, or a combination thereof in a patient. This method involves obtaining a small volume blood sample from a patient, determining the level of at least one immunosuppressant drug in the small volume blood sample, and determining the level of a second immunosuppressant drug or analyzing the renal function, the hepatic function, or a combination thereof in the small volume blood sample.

EP2533768B1 discloses methods for monitoring subject compliance with a prescribed treatment regimen. In one embodiment, the method involves measuring a drug level in a subject's fluid and normalizing the measured drug level as a function of one or more parameters associated with the subject. The drug concentration could be measured using LC-MS-MS or GC-MS-MS. The normalized drug level is compared to a reference value and associated confidence intervals or to a concentration range. The reference value and associated confidence intervals and/or the concentration range may be normalized based on one or more parameters associated with subjects in a reference population.

CN111458426A discloses a method for determining four types of benzodiazepines in blood. This method involves adding an extraction solvent to the plasma sample to obtain an extract, adding a composite adsorbent and a dewatering agent to the extraction liquid to perform purification and dewatering treatment to obtain a purified liquid, and after filtering the purified solution, it is detected and analyzed by LC-MS/MS.

### SUMMARY OF THE INVENTION

Given that antiarrhythmic drugs can only be used effectively within a narrow therapeutic window, it would be beneficial to develop a method to quantify them in a patient's blood. Furthermore, it would be advantageous to provide a method to allow the parallel determination of multiple antiarrhythmic drugs, which would expedite the analytical process.

The object of the invention is a method for monitoring a panel of antiarrhythmic drugs in a blood of a subject, the method comprising: obtaining a blood sample from the subject; determining in the blood sample, using liquid chromatography tandem mass spectrometry (LC-MS/MS), a level of at least one antiarrhythmic drug or antiarrhythmic drug metabolite selected from a group consisting of: Amiodarone, Propafenone, Sotalol, Digoxin, Ramipril, Eplerenone, Desetyl-Amiodarone, Hydroxy-Propafenone, Spironolactone, Metoprolol, Perindopril, Carvedilol, Bisoprolol and Nebivolol.

The blood sample can be a venous blood sample.

The blood sample is a capillary blood sample. The capillary blood sample can be collected on a sorbent. The method may further comprise converting the antiarrhythmic drug level in capillary blood to the antiarrhythmic drug level in venous blood by multiplying the antiarrhythmic drug level in capillary blood by a predetermined V/C ratio.

The invention also relates to a computer-implemented system for monitoring a panel of antiarrhythmic drugs in a blood of a subject, the system comprising: a liquid chromatography tandem mass spectrometry (LC-MS/MS) apparatus configured to receive a blood sample and to perform LC-MS/MS analysis to output LS-MS/MS measurement data; and an analytical module configured to receive the LS-MS/MS measurement data, the analytical module comprising at least one nontransitory processor-readable storage medium that stores at least one of processor-executable instructions and data and at least one processor communicably coupled to the storage medium and configured to, by executing the instructions and based on the data, determine in the blood sample a level of at least one antiarrhythmic drug or antiarrhythmic drug metabolite selected from a group consisting of: Amiodarone, Propafenone, Sotalol, Digoxin, Ramipril, Eplerenone, Desetyl-Amiodarone, Hydroxy-Propafenone, Spironolactone, Metoprolol, Perindopril, Carvedilol, Bisoprolol and Nebivolol.

### BRIEF DESCRIPTION OF THE DRAWINGS

The invention is shown by means of example embodiments described below and shown in a drawing, wherein:
Fig. 1 shows an example of chromatographic separation of 15 antiarrhythmic drugs (10 µg/L);
Fig. 2 shows a computer-implemented system for performing the method.

### DETAILED DESCRIPTION OF THE INVENTION

While the present invention can be embodied in various forms, the following description of several embodiments is provided with the understanding that the current disclosure is an exemplification of the invention. It is not intended to limit the invention to the specific embodiments illustrated. Headings are provided solely for convenience and should not be construed as limiting the invention in any manner. Embodiments illustrated under any heading may be combined with embodiments illustrated under any other heading.

The method according to the present invention involves measuring a concentration of two or more antiarrhythmic drugs belonging to a panel of antiarrhythmic drugs in a blood of a subject using liquid chromatography tandem mass spectrometry (LC-MS/MS), wherein the drugs are selected from a group comprising Amiodarone, Propafenone, Sotalol, Digoxin, Ramipril, Eplerenone, Desetyl-Amiodarone, Hydroxy-Propafenone, Spironolactone, Metoprolol, Perindopril, Carvedilol, Bisoprolol, Nebivolol.

The term "drug" as used herein refers to an active pharmaceutical ingredient and its metabolites, decomposition products, enantiomers, diastereomers, derivatives, etc.

Terms "subject", "patient", "individual" as used herein are used interchangeably and refer to a human individual who takes antiarrhythmic drugs.

The use of LC-MS/MS to determine blood levels of antiarrhythmic drugs allows for precise, quantitative determination of the concentration of these drugs. This information is crucial in therapeutic drug monitoring (TDM). Unlike immunological methods, the method presented here allows for simultaneous determination of the concentration of several drugs. This is important because the treatment of cardiac arrhythmias often involves a combination of several drugs with different mechanisms of action to achieve the desired therapeutic effect.

According to the present invention, LC-MS/MS can be used to determine the blood concentration of a panel of antiarrhythmic drugs, wherein the panel contains at least one antiarrhythmic drug, at least two antiarrhythmic drugs, at least three antiarrhythmic drugs, at least four antiarrhythmic drugs, at least five antiarrhythmic drugs, at least six antiarrhythmic drugs, at least seven antiarrhythmic drugs, at least eight antiarrhythmic drugs, at least nine antiarrhythmic drugs, at least ten antiarrhythmic drugs, at least eleven antiarrhythmic drugs, at least twelve antiarrhythmic drugs, at least thirteen antiarrhythmic drugs or at least fourteen antiarrhythmic drugs selected from the group comprising Amiodarone, Propafenone, Sotalol, Digoxin, Ramipril, Eplerenone, Desetyl-Amiodarone, Hydroxy-Propafenone, Spironolactone, Metoprolol, Perindopril, Carvedilol, Bisoprolol, Nebivolol.

In order to reduce the influence of the matrix effect on the final result of the drug concentration measurement and to ensure high accuracy, internal standards are used in the method of the invention. Internal standards (ISTDs) are isotopically labelled chemical compounds with similar characteristics to their corresponding analytes, in this case antiarrhythmic drugs. Isotopic labelling can be carried out with, for example, deuterium, C¹⁴, O¹⁸, N¹⁵.

Preferably, the internal standard for a particular drug takes the form of an isotopically labelled analogue of that drug (e.g. deuterium-labelled amiodarone). If no directly corresponding isotopically labelled analogue is available for a particular drug, another analogue similar in characteristics and retention time may be used instead (e.g. deuterium-labelled Spironolactone may be used for Zofenopril). One ISTD can correspond to more than one drug.

In some embodiments, at least one ISTD, at least two ISTDs, at least three ISTDs, at least four ISTDs, at lest five ISTDs, at least six ISTDs, at least seven ISTDs, at least eight ISTDs, at least nine ISTDs or at least ten ISTDs can be used, selected from a group comprising Sotalol-d6, Perindopril-d4, Propafenone-d5, Carvedilol-d3, Nebivolol-d4, Digoxin-d3, Spironolactone-d6, Desethylamiodarone-d4, Amiodarone-d4 and Dronedarone-d6.

The use of a plurality of ISTDs in the LC-MS/MS analysis allows for a more accurate measurement and the inclusion of more drugs in a single analysis. This allows a single sample to be analyzed for multiple drugs more quickly.

In some embodiments, the blood sample being analyzed may be venous blood. The venous blood sample may be analyzed as whole blood, or it may be separated into individual fractions and the plasma or serum may be analyzed.

In some embodiments, the blood sample being analyzed may be capillary blood. The capillary blood sample can be taken by the patient whose blood will be tested on their own and sent to a laboratory for further analysis. Alternatively, the blood sample can be taken by a medical practitioner (e.g. a doctor or nurse). The capillary blood sample may be collected on a sorbent, blotting paper or another type of a dry capillary blood collection kit. Such a sample may also be referred to as a dried blood spot (DBS).

The use of capillary blood for testing allows for a simplified sampling process. By allowing the patient to take the sample themselves, the test can be carried out without a need for the patient having to visit an outpatient clinic or a sampling center.

Blood samples are processed in a manner suitable for preparation for LC-MS/MS analysis to determine the concentration of antiarrhythmic drugs. However, this method is not discussed in detail as it is not the object of the invention and is obvious to a specialist in LC-MS/MS analysis.

In some embodiments, the determination of antiarrhythmic drug levels by LC-MS/MS is performed on capillary blood, and the resulting capillary blood drug concentration is then converted to the corresponding venous blood drug concentration. This conversion is performed by multiplying the capillary blood drug concentration by the ratio of the venous blood drug concentration to the capillary blood drug concentration (V/C ratio).

The V/C ratio can be determined experimentally by correlating drug concentrations in venous blood and capillary blood in the same patient at a given time point. By determining this correlation, it is possible to make practical use of the data obtained on the concentration of a particular drug in the patient's capillary blood, as therapeutic standards are set only for drug concentrations in venous blood. V/C ratios for specific drugs are shown in Table 1.

**Table 1. V/C ratios**

| **Drug** | **V/C ratio** | **Precision [%]** |
|---|---|---|
| Amiodarone | 2.10 | 2.95 |
| Propafenone | 1.82 | 9.88 |
| Sotalol | 0.94 | 6.05 |
| Digoxin | 0.69 | 1.22 |
| Ramipril | 1.15 | 7.90 |
| Eplerenone | 1.78 | 5.12 |
| Desethyl-Amiodarone | 1.28 | 12.43 |
| Hydroxy-Propafenone | 1.00 | 7.33 |
| Spironolactone | 1.76 | 5.19 |
| Metoprolol | 0.87 | 9.79 |
| Perindopril | 1.86 | 4.91 |
| Carvedilol | 1.86 | 4.89 |
| Bisoprolol | 1.06 | 4.35 |
| Nebivolol | 1.04 | 1.71 |

The correlation for all drugs listed in the table was statistically significant.

In some embodiments presented below, the antiarrhythmic drugs taken by the patient were determined before the blood sample was submitted to the analytical laboratory. This could have been done by taking a medical history, the patient completing a questionnaire or a form provided with the blood sample kit. Based on this information, only ISTDs corresponding to the drugs taken by the patient were utilized at the LC-MS/MS analysis stage.

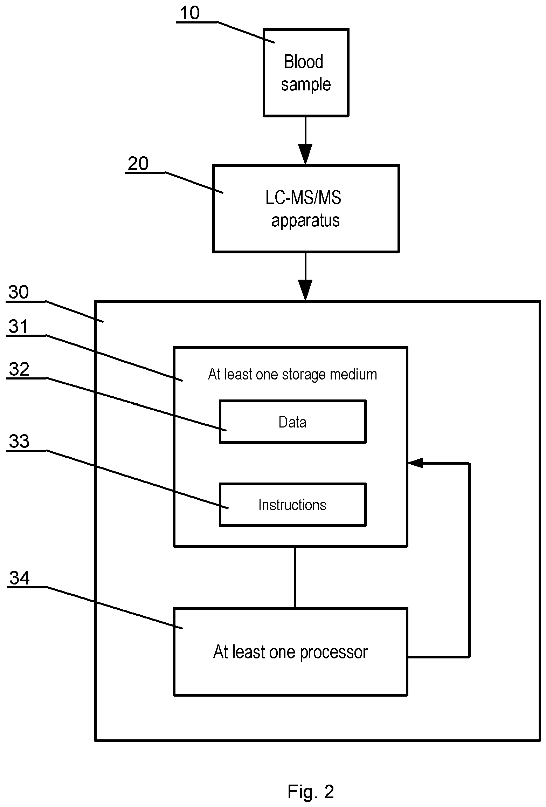

The method described above can be implemented in a computer-implemented system, such as shown in Fig. 2. An LC-MS/MS apparatus 20 is configured to receive a blood sample 10 and to perform the LC-MS/MS analysis to output typical LS-MS/MS measurement data, such as a chromatogram, mass spectrum analysis etc. Any standard LC-MS/MS system can be used for that purpose. The output data is fed to an analytical module 30 that can be a module embedded in the LC-MS/MS apparatus and appropriately configured as described herein or an external module. The analytical module 30 contains at least one nontransitory processor-readable storage medium 31 that stores at least one of processor-executable instructions 33 and data 32 (such as the output data from the LC-MS/MS analysis, V/C ratios etc.), and at least one processor 34 communicably coupled to the storage medium 31. The at least one processor 34 is configured to (by executing the instructions 33 and based on the data 32) determine in the blood sample a level of at least one antiarrhythmic drug or antiarrhythmic drug metabolite selected from a group consisting of: Amiodarone, Propafenone, Sotalol, Digoxin, Ramipril, Eplerenone, Desetyl-Amiodarone, Hydroxy-Propafenone, Spironolactone, Metoprolol, Perindopril, Carvedilol, Bisoprolol and Nebivolol, according to the details of the method described herein.

### EXAMPLES

### Example 1. A targeted LC-MS/MS analytical method

The developed and optimized LC-MS/MS method included the determination of creatinine (by test) and 15 antiarrhythmic drugs against 3 internal standards. The best chromatographic separation parameters were obtained on an Agilent InfinityLab Poroshell 120 PFP column, 3.0 x 100 mm, 2.7 µm. The most important separation conditions included:
- Injection volume: 10.00 µl
- Phase A: Water supplemented with 0.1% FA and 2 mM AF
- Phase B: ACN:H₂O [95:5] with the addition of 0.1% FA and 2 mM AF
- Flow rate - 0.5 mL/min
- Gradient - 10%B up to 0.5 min, linear gradient to 100%B at 6 min, 100%B up to 8 min, 8.1 min - return to 10%B, end of analysis at 10 min.
- Column temperature - 45°C
- Autosampler temperature - 8°C

Due to the different values of the ionization parameters for the different compounds, it was decided to divide the method into four scanning periods with different ion source parameters. A summary of the MS/MS parameters is presented in Table 2.

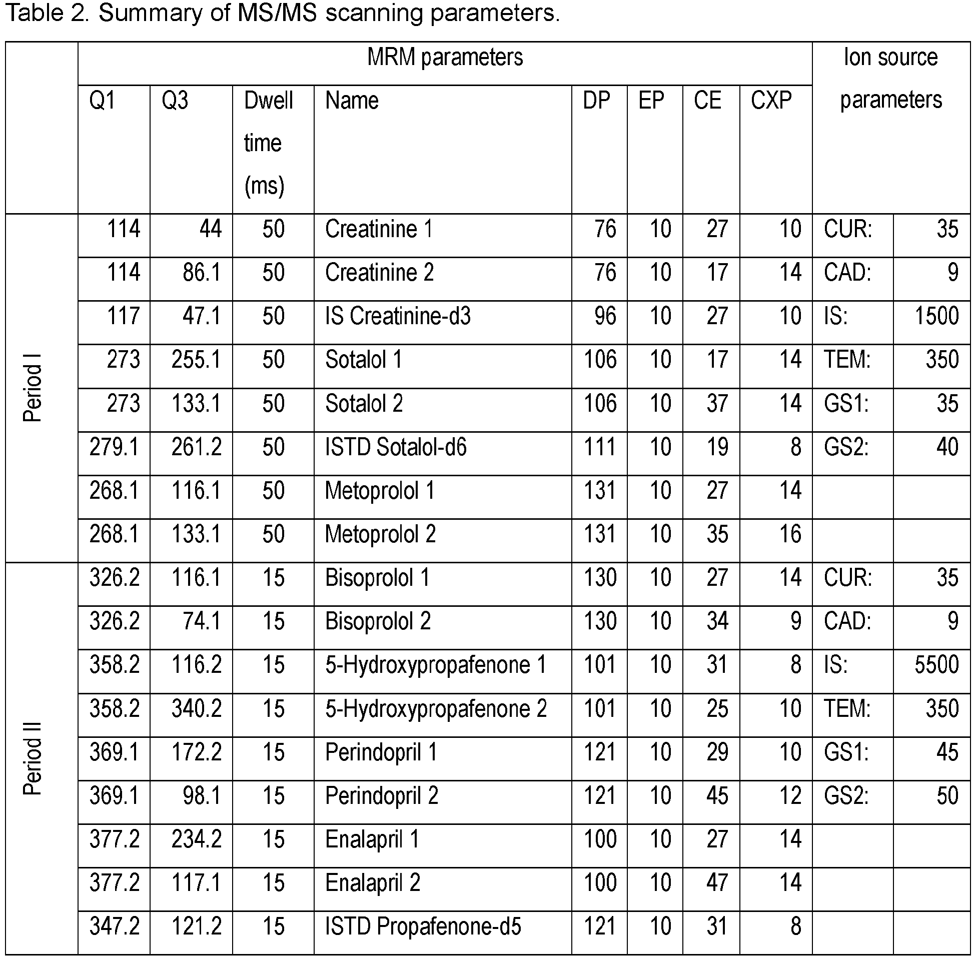

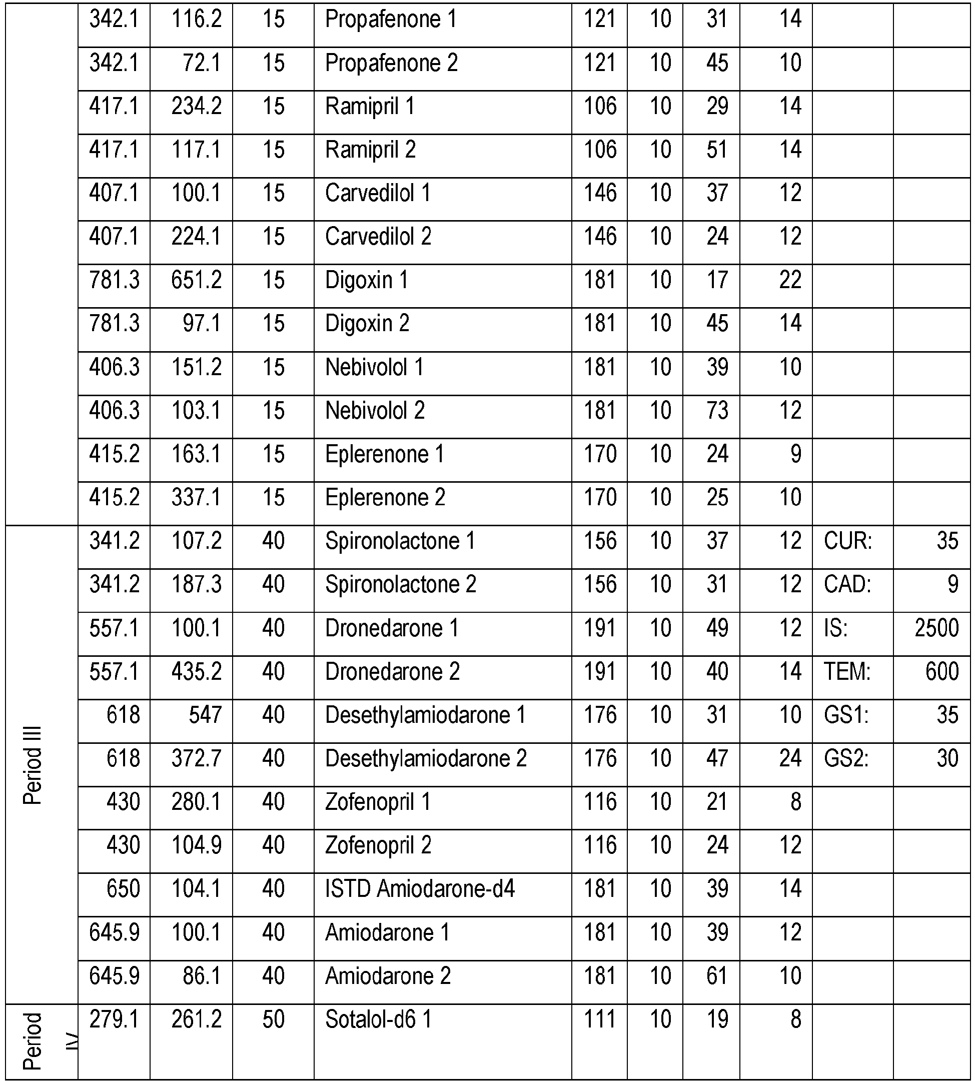

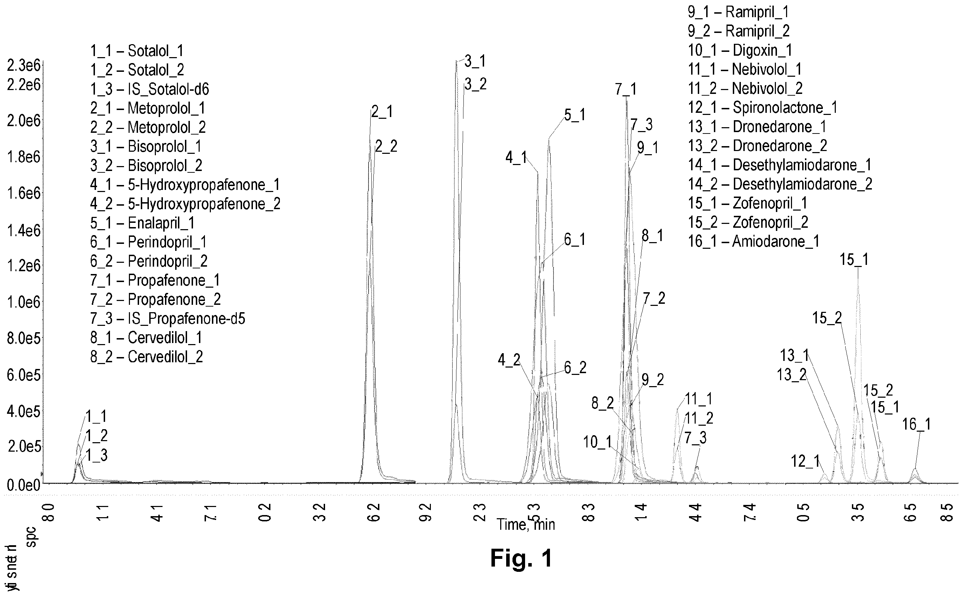

The scanning parameters allow the collection of 10 or more measurement points per chromatographic peak, the LOD (S/N≥3) for all compounds is less than or equal to 0.1 µg/L. An example chromatogram is shown in Fig.1. The optimized LC-MS/MS methodology outlined above was applied to serum and whole blood analyses.

### Example 2. Validation of a quantitative LC-MS/MS method for natural serum samples

Serum samples for standard curves, determination of recovery, precision and repeatability of the analytical procedure were fortified with test compounds and ISTD, and then prepared for analysis according to the following protocol:
a) Mix serum in a tube by pipetting several times.
b) Homogenise the serum by pipetting 500 µl into an Eppendorf tube (1.5 ml).
c) Add an appropriate amount of a compound (10 µl from each concentration) and an internal standard (ISTD) (10 µl from a concentration of 250 ng/ml resulting in a final sample concentration of 5 ng/ml).
d) Mix by vortexing (10-15s).
e) Perform de-proteinisation by adding 1000 µl ACN acidified with 0.1% FA. After adding ACN, vortex the sample as quickly as possible (time: 1 min).
f) Centrifuge for 10 min at 15,000 rpm on a mini-centrifuge (Benchmark Scientific In.).
g) After centrifugation, carefully remove all supernatant and transfer to a clean chromatography vial.
h) Evaporate using an UltraVap Levante concentrator (Porvair Sciences) - 60 min, 50°C.
i) After evaporation, dissolve in vol. 500 µl of starter phase (10% ACN; 2 mM AF; 0.1% FA).
j) Vortex for 1 min.
k) Transfer to an Eppendorf type tube (1.5 ml) and centrifuge on a mini-centrifuge for 10 min (15000 rpm).
l) Transfer the supernatant to a 350 µl insert.
m) Perform LC-MS/MS analysis.

The obtained results are shown in the tables below.

**Table 3. LOD, LOQ, R, Operating range (linearity)**

| *Drug* | *Ion ratio* | *LOD (S*/*N*≥*3)* | | *LOQ (S*/*N*≥*6)* | | *Regression coefficient (R≥0.995)* | *Linearity range [µg*/*L]* |
|---|---|---|---|---|---|---|---|
| | | *µg*/*L* | *S*/*N* | *µg*/*L* | *S*/*N* | | |
| *Sotalol* | 0.573 | 0.1 | 4.2 | 0.25 | 7.8 | 0.99804 | 0.25 - 100 |
| *Metoprolol* | 0.637 | 0.1 | 268 | 0.1 | 268 | 0.99737 | 0.10 - 25 |
| *Bisoprolol* | 0.186 | 0.1 | 287 | 0.1 | 287 | 0.99815 | 0.10 - 100 |
| *5-OH Propafenone* | 0.280 | 0.1 | 183 | 0.1 | 183 | 0.99905 | 0.10 - 100 |
| *Perindopril* | 0.478 | 0.1 | 125 | 0.1 | 125 | 0.99881 | 0.10 - 100 |
| *Enalapril* | 0.299 | 0.1 | 92 | 0.1 | 92 | 0.99857 | 0.10 - 100 |
| *Propafenone* | 0.294 | 0.1 | 220 | 0.1 | 220 | 0.99884 | 0.10 - 100 |
| *Ramipril* | 0.250 | 0.1 | 139 | 0.1 | 139 | 0.99951 | 0.10 - 100 |
| *Carvedilol* | 0.387 | 0.1 | 87 | 0.1 | 87 | 0.99931 | 0.10 - 100 |
| *Digoxin* | 0.549 | 0.1 | 42 | 0.1 | 42 | 0.99923 | 0.10 - 100 |
| *Nebivolol* | 0.485 | 0.1 | 19 | 0.1 | 19 | 0.99889 | 0.10 - 100 |
| *Eplerenone* | 0.306 | 0.1 | 11 | 0.1 | 11 | 0.99895 | 0.10 - 100 |
| *Spironolactone* | 0.707 | 0.1 | 12 | 0.1 | 12 | 0.99912 | 0.10 - 100 |
| *Dronedarone* | 0.571 | 0.1 | 33 | 0.1 | 33 | 0.99934 | 0.10 - 100 |
| *Desethylamiodarone* | 0.329 | 0.1 | 130 | 0.1 | 130 | 0.99966 | 0.10 - 100 |
| *Zofenopril* | 0.621 | 0.1 | 14 | 0.1 | 14 | 0.99908 | 0.10 - 100 |
| *Amiodarone* | 0.355 | 0.1 | 16 | 0.1 | 16 | 0.99821 | 0.10 - 100 |

**Table 4. Recovery**

| Drug | Recovery serum [%] | | |
|---|---|---|---|
| | Low (0.5 µg/L) | Medium (2.5 µg/L) | High (20 µg/L) |
| Sotalol | 102.53 | 109.79 | 104.30 |
| Metoprolol | 96.70 | 109.61 | 106.41 |
| Bisoprolol | 95.53 | 107.18 | 109.41 |
| 5-OH Propafenone | 89.32 | 105.83 | 106.29 |
| Perindopril | 90.06 | 101.69 | 102.61 |
| Enalapril | 92.47 | 101.49 | 100.58 |
| Propafenone | 95.82 | 105.02 | 103.68 |
| Ramipril | 93.81 | 106.00 | 104.78 |
| Carvedilol | 88.48 | 99.93 | 97.56 |
| Digoxin | 97.77 | 117.61 | 111.59 |
| Nebivolol | 85.28 | 98.12 | 91.24 |
| Eplerenone | 83.98 | 107.77 | 104.01 |
| Spironolactone | 85.42 | 93.36 | 103.19 |
| Dronedarone | 98.71 | 100.68 | 95.72 |
| Desethylam iodarone | 112.86 | 101.97 | 98.05 |
| Zofenopril | 81.09 | 95.68 | 91.77 |
| Amiodarone | 92.89 | 101.04 | 103.26 |

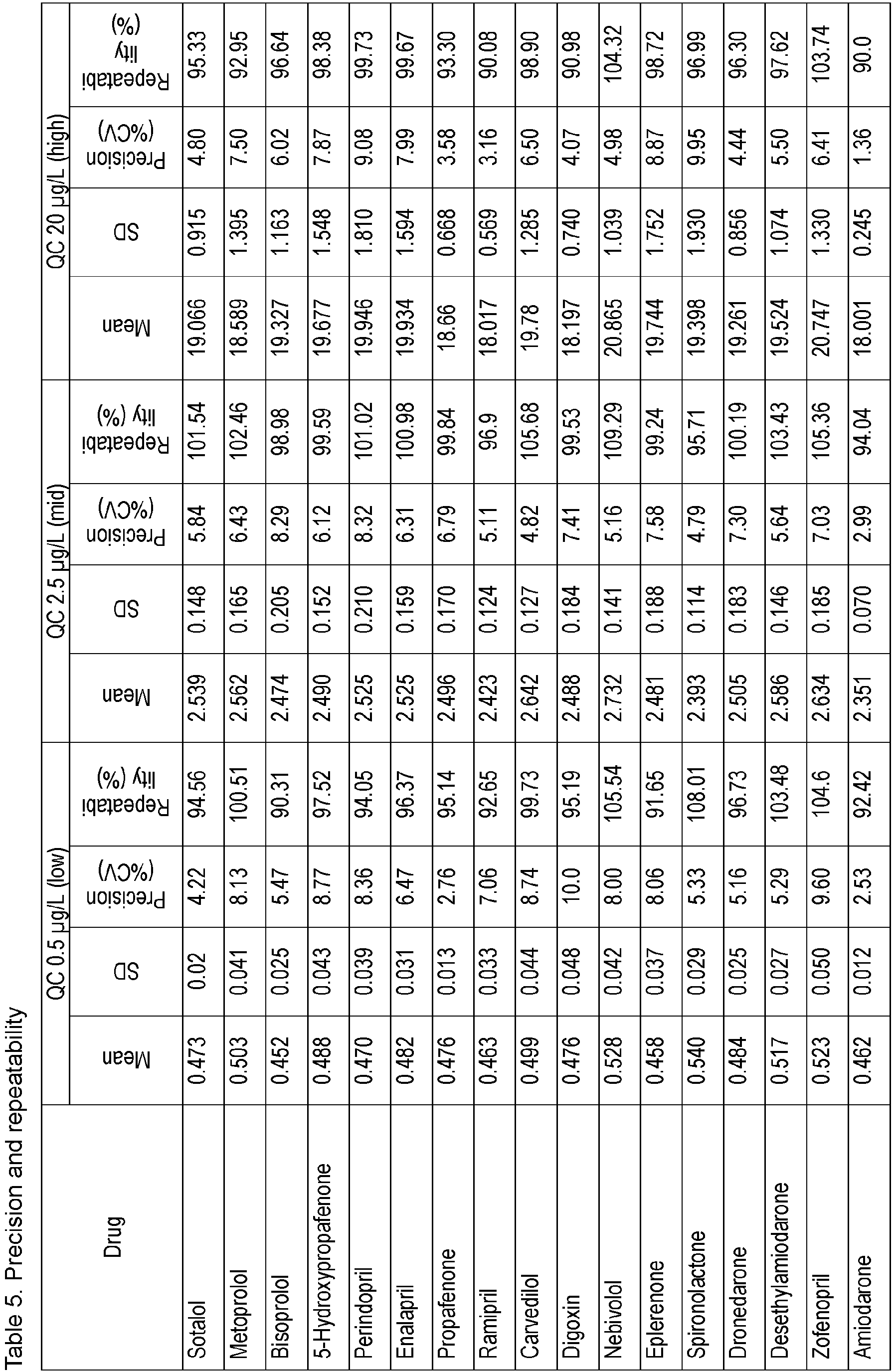

### Example 3. Validation of a quantitative LC-MS/MS method for venous blood samples

Whole blood samples for standard curves, determination of recovery, precision and repeatability of the analytical procedure were fortified with test compounds and ISTD and then prepared for analysis according to the following protocol:
a) Mix blood in a tube by pipetting several times.
b) Homogenise blood in a volume of 200 µl into an Eppendorf-type tube (1.5 ml).
c) Add appropriate amount of compounds (10 µl each from the appropriate concentration) and ISTD (10 µl from a concentration of 100 ng/ml giving a final concentration of 5 ng/ml in the sample).
d) Mix by vortexing (10-15s).
e) Incubate 5 min at room temperature.
f) Perform deproteinisation by adding 600 µl ACN acidified with 0.1% FA. After adding ACN, vortex the sample as quickly as possible (time: 1 min).
g) Centrifuge on a mini-centrifuge (Benchmark Scientific Inc.) for 10 min at 15000 rpm.
h) After centrifugation, carefully remove all supernatant and transfer to a clean chromatography vial.
i) Evaporate using an UltraVap Levante concentrator (Porvair Sciences) - 50 min, 50°C.
j) After evaporation, dissolve in vol. 200 µl of starter phase (10% ACN; 2 mM AF; 0.1% FA).
k) Vortex for 1 min.
l) Sonicate on an ultrasonic cleaner for 5 min.
m) Transfer to an Eppendorf type tube (1.5 ml) and centrifuge for 10 min (15000 rpm).
n) Transfer the supernatant to a 250 µl insert.
o) LC-MS/MS analysis.

The obtained results are shown in the tables below.

**Table 6. LOD, LOQ, R, Operating range (linearity)**

| *Drug* | *Ion ratio* | *LOD (S*/*N≥3)* | | *LOQ (S*/*N≥6)* | | *Regression coefficient (R≥0.995)* | *Linearity range [µg*/*L]* |
|---|---|---|---|---|---|---|---|
| | | *µg*/*L* | *S*/*N* | *µg*/*L* | *S*/*N* | | |
| *Sotalol* | 0.566 | 0.1 | 3.7 | 0.25 | 7.2 | 0.99963 | 0.25-100 |
| *Metoprolol* | 0.627 | 0.1 | 99 | 0.1 | 99 | 0.99941 | 0.10 - 50 |
| *Bisoprolol* | 0.195 | 0.1 | 84 | 0.1 | 84 | 0.99803 | 0.10 - 100 |
| *5-OH Propafenone* | 0.279 | 0.1 | 96 | 0.1 | 96 | 0.99788 | 0.10 - 100 |
| *Perindopril* | 0.487 | 0.1 | 83 | 0.1 | 83 | 0.99738 | 0.10 - 100 |
| *Enalapril* | 0.294 | 0.1 | 28 | 0.1 | 28 | 0.99724 | 0.10 - 100 |
| *Propafenone* | 0.300 | 0.1 | 99 | 0.1 | 99 | 0.99823 | 0.10 - 100 |
| *Ramipril* | 0.248 | 0.1 | 197 | 0.1 | 197 | 0.99902 | 0.10 - 100 |
| *Carvedilol* | 0.353 | 0.1 | 48 | 0.1 | 48 | 0.99869 | 0.10 - 100 |
| *Digoxin* | 0.664 | 0.1 | 3.3 | 0.25 | 10.7 | 0.99754 | 0.25 - 100 |
| *Nebivolol* | 0.486 | 0.1 | 17.5 | 0.1 | 17.5 | 0.99695 | 0.10 - 50 |
| *Eplerenone* | 0.360 | 0.1 | 5 | 0.25 | 14 | 0.99791 | 0.25 - 100 |
| *Spironolactone* | 0.666 | 0.1 | 9.3 | 0.1 | 9.3 | 0.99825 | 0.10 - 100 |
| *Dronedarone* | 0.500 | 0.1 | 3.1 | 0.25 | 8.2 | 0.99734 | 0.25 - 100 |
| *Desethylamiodarone* | 0.319 | 0.25 | 5.8 | 0.5 | 24 | 0.9957 | 0.50 - 100 |
| *Zofenopril* | 0.547 | 0.1 | 46 | 0.1 | 46 | 0.99728 | 0.10 - 100 |
| *Amiodarone* | 0.405 | 0.1 | 4.2 | 0.25 | 27 | 0.99817 | 0.25 - 100 |

**Table 7. Recovery**

| Drug | Recovery blood [%] | | |
|---|---|---|---|
| | Low (0.5 µg/L) | Medium (2.5 µg/L) | High (20 µg/L) |
| Sotalol | 105.85 | 95.38 | 107.82 |
| Metoprolol | 108.23 | 96.65 | 104.24 |
| Bisoprolol | 111.23 | 99.08 | 109.02 |
| 5-OH Propafenone | 104.62 | 87.66 | 106.77 |
| Perindopril | 99.46 | 89.00 | 109.57 |
| Enalapril | 100.57 | 86.61 | 116.60 |
| Propafenone | 114.03 | 92.52 | 106.43 |
| Ramipril | 102.92 | 87.64 | 104.82 |
| Carvedilol | 89.14 | 91.21 | 105.64 |
| Digoxin | 117.19 | 87.31 | 99.99 |
| Nebivolol | 80.64 | 88.99 | 102.58 |
| Eplerenone | 119.71 | 89.65 | 95.48 |
| Spironolactone | 103.49 | 93.52 | 93.47 |
| Dronedarone | 82.67 | 80.55 | 80.13 |
| Desethylam iodarone | 104.57 | 108.11 | 116.31 |
| Zofenopril | 80.30 | 80.01 | 86.06 |
| Amiodarone | 103.02 | 91.57 | 106.02 |

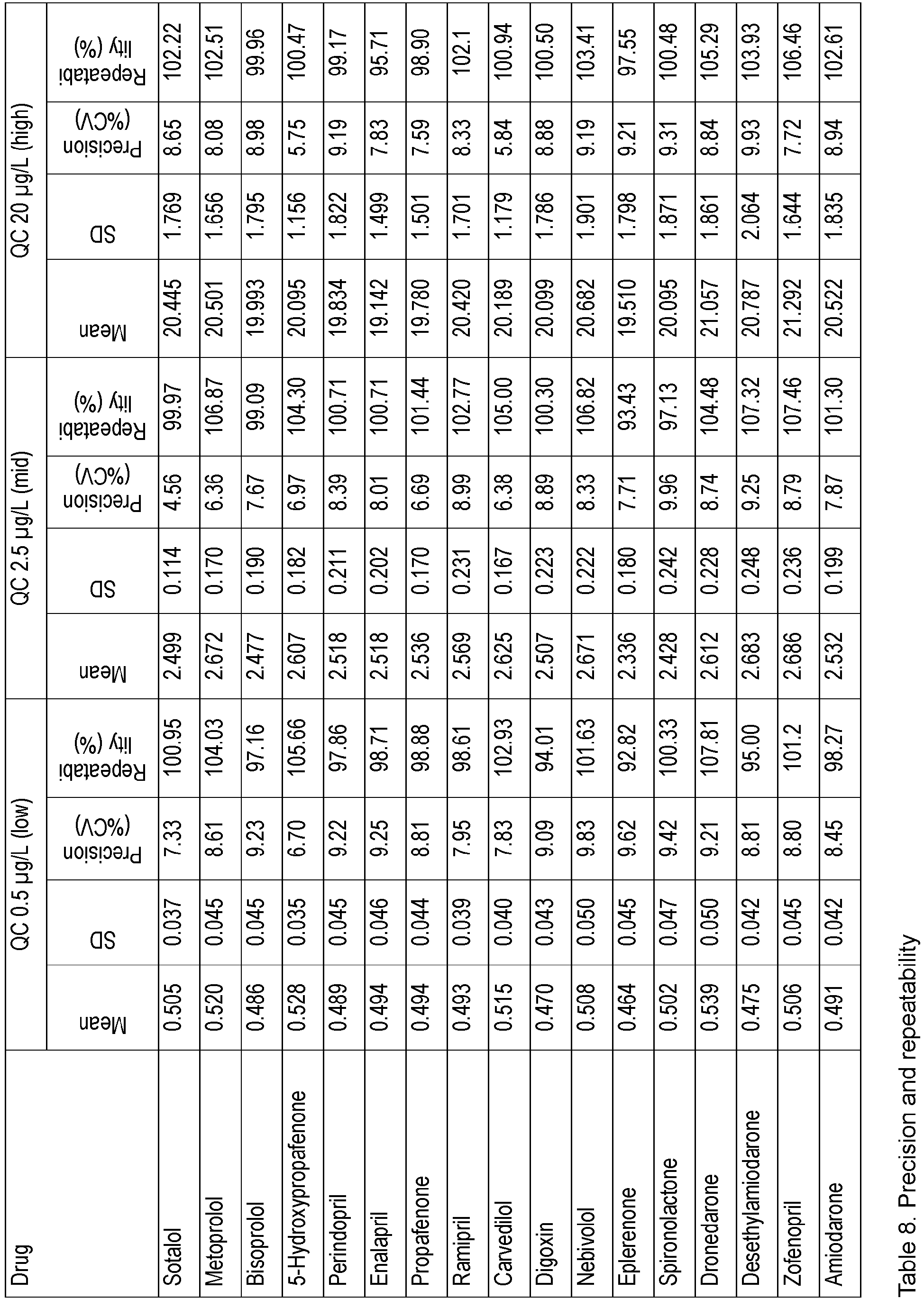

### Example 4. Optimized LC-MS/MS method for capillary blood

The optimised LC-MS/MS method for capillary blood collected on sorbent included the determination of 15 antiarrhythmic drugs against 10 internal standards. The best chromatographic separation parameters were obtained on an Agilent InfinityLab Poroshell 120 PFP column, 3.0 x 100 mm, 2.7 µm. Key separation conditions included:
- Injection volume: 10.00 µl
- Phase A: Water with 0.1% FA
- Phase B: ACN with the addition of 0.1% FA
- Flow rate - 0.5 mL/min
- Gradient - 5%B up to 0.5 min, linear gradient to 40%B in 2 min, linear gradient to 60%B in 3.9 min, 95%B from 4 to 6 min, linear gradient to 98% B in 8 min, 8.1 min, - return to 5%B, end of analysis in 10 min.
- Column temperature - 45°C
- Autosampler temperature - 8°C

Due to the different values of the ionisation parameters for the different compounds, it was decided to divide the method into five scanning periods with different ion source parameters and advanced MS/MS scanning settings. A summary of the MS/MS parameters is presented in Table 9.

**Table 9. Summary of MS/MS scanning parameters.**

| Period | MRM parameters | | | | | | | | Ion source parameters | |
|---|---|---|---|---|---|---|---|---|---|---|
| | Q1 | Q3 | Dwell time (ms) | Name | DP | EP | CE | CXP | | |
| I | 273 | 255.1 | 100 | Sotalol 1 | 106 | 10 | 17 | 14 | CUR: | 35 |
| | 273 | 133.1 | 100 | Sotalol 2 | 106 | 10 | 37 | 14 | CAD: | 9 |
| | 279.1 | 261.2 | 100 | ISTD Sotalol-d6 | 111 | 10 | 19 | 8 | IS: | 1500 |
| | | | | | | | | | TEM: | 350 |
| | | | | | | | | | GS1: | 35 |
| | | | | | | | | | GS2: | 40 |
| II | 326.2 | 116.1 | 50 | Bisoprolol 1 | 130 | 10 | 27 | 14 | CUR: | 35 |
| | 326.2 | 74.1 | 50 | Bisoprolol 2 | 130 | 10 | 34 | 9 | CAD: | 9 |
| | 268.1 | 116.1 | 50 | Metoprolol 1 | 131 | 10 | 27 | 14 | IS: | 5500 |
| | 268.1 | 133.1 | 50 | Metoprolol 2 | 131 | 10 | 35 | 16 | TEM: | 350 |
| | | | | | | | | | GS1: | 45 |
| | | | | | | | | | GS2: | 50 |
| III | 377.2 | 234.2 | 20 | Enalapril 1 | 100 | 10 | 27 | 14 | jw. | |
| | 377.2 | 117.1 | 20 | Enalapril 2 | 100 | 10 | 47 | 14 | | |
| | 358.2 | 116.2 | 20 | 5-Hydroxypropafenone 1 | 101 | 10 | 31 | 8 | | |
| | 358.2 | 340.2 | 20 | 5-Hydroxypropafenone 2 | 101 | 10 | 25 | 10 | | |
| | 417.1 | 234.2 | 20 | Ramipril 1 | 106 | 10 | 29 | 14 | | |
| | 417.1 | 117.1 | 20 | Ramipril 2 | 106 | 10 | 51 | 14 | | |
| | 373.2 | 176.2 | 20 | IS Perindopril-d4 | 111 | 10 | 29 | 10 | | |
| | 369.1 | 172.2 | 20 | Perindopril 1 | 121 | 10 | 29 | 10 | | |
| | 369.1 | 98.1 | 20 | Perindopril 2 | 121 | 10 | 45 | 12 | | |
| | 347.2 | 121.2 | 20 | IS Propafenone-d5 | 121 | 10 | 31 | 8 | | |
| | 342.1 | 116.2 | 20 | Propafenone 1 | 121 | 10 | 31 | 14 | | |
| | 342.1 | 72.1 | 20 | Propafenone 2 | 121 | 10 | 45 | 10 | | |
| | 410.1 | 100.1 | 20 | IS Carvedilol-d3 | 126 | 10 | 39 | 12 | | |
| | 410.1 | 151.1 | 20 | IS Nebivolol-d4 | 141 | 10 | 41 | 18 | | |
| | 407.1 | 100.1 | 20 | Carvedilol 1 | 146 | 10 | 37 | 12 | | |
| | 407.1 | 224.1 | 20 | Carvedilol 2 | 146 | 10 | 24 | 12 | | |
| | 406.3 | 151.2 | 20 | Nebivolol 1 | 150 | 10 | 39 | 10 | | |
| | 406.3 | 103.1 | 20 | Nebivolol 2 | 150 | 10 | 73 | 12 | | |
| IV | 781.3 | 651.2 | 100 | Digoxin 1 | 140 | 10 | 16 | 21 | jw. | |
| | 781.3 | 97.1 | 100 | Digoxin 2 | 140 | 10 | 44 | 14 | | |
| | 784.4 | 654.4 | 100 | IS Digoxin-d3 | 140 | 10 | 17 | 12 | | |
| V | 430 | 280.1 | 20 | Zofenopril 1 | 116 | 10 | 21 | 8 | CUR: | 35 |
| | 430 | 104.9 | 20 | Zofenopril 2 | 116 | 10 | 24 | 12 | CAD: | 9 |
| | 347.3 | 107.1 | 20 | IS Spironolactone-d6 | 156 | 10 | 39 | 12 | IS: | 2500 |
| | 341.2 | 107.2 | 20 | Spironolactone 1 | 156 | 10 | 37 | 12 | TEM: | 600 |
| | 341.2 | 187.3 | 20 | Spironolactone 2 | 156 | 10 | 31 | 12 | GS1: | 35 |
| | 415.2 | 163.1 | 20 | Eplerenone 1 | 170 | 10 | 24 | 9 | GS2: | 30 |
| | 415.2 | 337.1 | 20 | Eplerenone 2 | 170 | 10 | 25 | 10 | | |
| | 621.8 | 372.8 | 20 | IS Desethylamiodarone-d4 | 171 | 10 | 46 | 28 | | |
| | 618 | 547 | 20 | Desethylamiodarone 1 | 176 | 10 | 31 | 10 | | |
| | 618 | 372.7 | 20 | Desethylamiodarone 2 | 176 | 10 | 47 | 24 | | |
| | 650 | 104.1 | 20 | IS Amiodarone-d4 | 181 | 10 | 39 | 14 | | |
| | 645.9 | 100.1 | 20 | Amiodarone 1 | 181 | 10 | 39 | 12 | | |
| | 645.9 | 276.2 | 20 | Amiodarone 2 | 181 | 10 | 51 | 10 | | |
| | 563.2 | 441.3 | 20 | IS Dronedarone-d6 | 171 | 10 | 46 | 8 | | |
| | 557.1 | 100.1 | 20 | Dronedarone 1 | 191 | 10 | 49 | 12 | | |
| | 557.1 | 435.2 | 20 | Dronedarone 2 | 191 | 10 | 40 | 14 | | |
| VI | 279.1 | 261.2 | 50 | Sotalol-d6 | 111 | 10 | 19 | 8 | | |

The scanning parameters allow collecting 10 or more measurement points per chromatographic peak. The optimized LC-MS/MS methodology outlined above could be used for capillary blood, serum and whole blood analyses.

### Example 5. Validation of a quantitative LC-MS/MS method for capillary blood samples

Capillary blood samples for standard curves, determination of recovery, precision and repeatability of the analytical procedure were fortified with test compounds and ISTD and then prepared for analysis according to the following protocol:
a) Collecting 980 µl of homogenised blood into a 1.5 ml Eppendorf type tube.
b) Spike: Adding appropriate working solution (20 µl)
c) Shaking and incubation (Vortex, 10 min)
d) Transferring spiked blood to petri dish
e) Collecting blood on sorbent
f) Drying at room temperature: 4 hours
g) Transferring dried sorbents into Eppendorf tubes: 2 ml
h) Adding 100 µl H₂O + 0.1% FA
i) Performing sonication on an ultrasonic cleaner: 10 min, 37°C
j) Adding 400 µl ACN + internal standard mixture -> shaking 20 min (vortex)
k) Performing centrifugation (16000 rpm, 10 min)
l) Collecting supernatant and transferring to chromatographic vials
m) Evaporating under nitrogen (50°C, 30 min)
n) Performing dissolution in 5% ACN+0.1% FA: 100 µl (shaking 2 min)
o) Performing sonication (5 min, room temperature)
p) Extracting all liquid and transferring to new Eppendorf tubes: 1.5 ml
q) Performing centrifugation (16000 rpm, 10 min)
r) Collecting supernatant and transferring to inserts, performing LC-MS/MS analysis

The obtained results are shown in the tables below.

**Table 10. LOD, LOQ, R, Operating range (linearity)**

| Drug | *Regression coefficient* (R) | Linearity range [µg/L] | LOD [µg/L] | LOQ [µg/L] | Signal to Noise |
|---|---|---|---|---|---|
| Sotalol | r = 0.99806 | 10 - 2500 | < 10 | <10 | 3406 |
| Metoprolol | r = 0.99974 | 1 - 250 | < 1 | < 1 | 261 |
| Bisoprolol | r = 0.99917 | 2.5 - 250 | < 1 | < 1 | 438 |
| 5-OH Propafenone | r = 0.99875 | 50 -2500 | <10 | < 10 | 2677 |
| Perindopril | r = 0.99697 | 10 - 250 | < 1 | < 1 | 811 |
| Enalapril | r = 0.99504 | 2.5 - 250 | < 1 | < 1 | 561 |
| Propafenone | r = 0.99704 | 1 - 250 | < 1 | < 1 | 1136 |
| Ramipril | r = 0.99869 | 2.5 - 250 | < 1 | < 1 | 435 |
| Carvedilol | r = 0.99628 | 2.5 - 250 | < 1 | < 1 | 298 |
| Digoxin | r = 0.99754 | 0.25 - 25 | < 0.25 | 0.25 | 7 |
| Nebivolol | r = 0.99749 | 0.25 - 25 | < 0.1 | 0.1 | 6 |
| Eplerenone | r = 0.99808 | 10 - 2500 | < 1 | < 1 | 95 |
| Spironolactone | r = 0.99851 | 2.5 - 250 | < 1 | < 1 | 33 |
| Dronedarone | r = 0.99805 | 2.5 -250 | < 1 | < 1 | 127 |
| Desethylamiodarone | r = 0.99688 | 10 -2500 | < 10 | < 10 | 456 |
| Zofenopril | r = 0.99744 | 2.5 - 250 | < 1 | < 1 | 57 |
| Amiodarone | r = 0.99726 | 10 - 2500 | <10 | <10 | 438 |

**Table 11. Recovery**

| Drug | Recovery [%] | | |
|---|---|---|---|
| | Low (0.5/5/50 µg/L) | Medium (2.5/25/250 µg/L) | High (10/100/1000 µg/L) |
| Sotalol | 102.17 | 116.45 | 98.82 |
| Metoprolol | 105.07 | 109.34 | 92.20 |
| Bisoprolol | 105.24 | 107.32 | 85.18 |
| 5-OH Propafenone | 96.39 | 113.65 | 96.25 |
| Perindopril | 102.83 | 114.50 | 95.65 |
| Enalapril | 98.02 | 109.50 | 88.35 |
| Propafenone | 98.42 | 89.39 | 80.54 |
| Ramipril | 101.96 | 88.75 | 95.66 |
| Carvedilol | 94.07 | 111.10 | 95.55 |
| Digoxin | 81.48 | 113.05 | 90.62 |
| Nebivolol | 93.71 | 110.78 | 92.06 |
| Eplerenone | 115.75 | 111.96 | 112.26 |
| Spironolactone | 100.26 | 108.36 | 92.76 |
| Dronedarone | 96.14 | 107.40 | 93.96 |
| Desethylam iodarone | 86.56 | 94.12 | 84.51 |
| Zofenopril | 111.93 | 104.88 | 99.05 |
| Amiodarone | 98.08 | 97.96 | 86.50 |

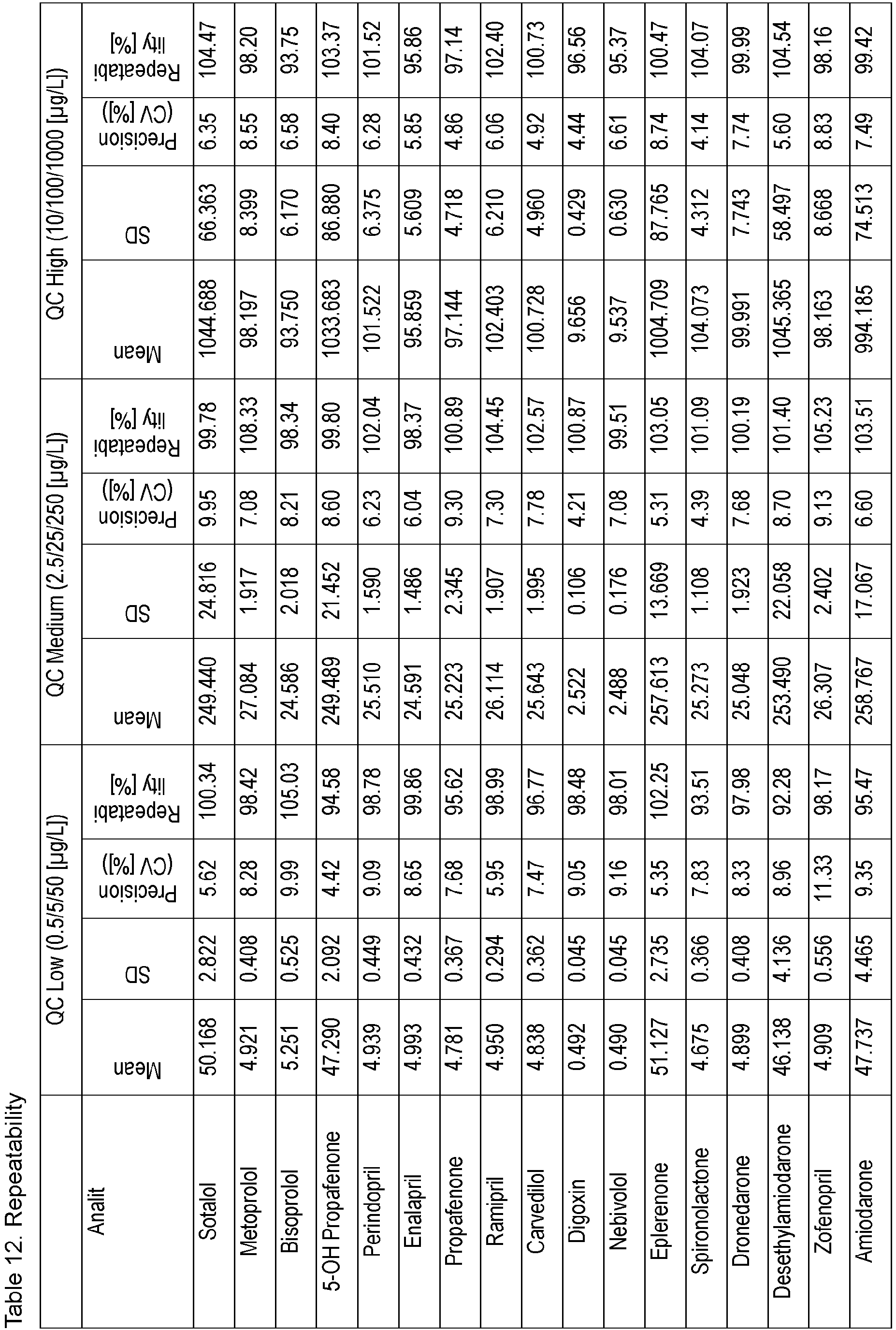

### Example 6. Quantitative analysis of antiarrhythmic drug concentrations in venous and capillary blood during therapy

During the clinical study of antiarrhythmic drug concentrations in venous and capillary blood, taking into account actual analytical repetitions, the following analyses were performed:
1159 analyses from venous blood (4 outpatient sampling)
10 551 analyses from capillary blood collected on a sorbent (4 outpatient + 9 home sampling)
To determine the reproducibility of the analytical procedure in capillary and venous blood, data obtained in independent studies performed in two laboratories were used for samples collected during medical visits at 0, 6, 12, 18 months of the study. The number of patients analyzed is summarized in Table 13. Reproducibility results are shown in Table 14.

**Table 13. Number of patients analyzed during medical visits at 0, 6, 12, 18 months of study.**

| | Sampling 1 | Sampling 2 | Sampling 3 | Sampling 4 |
|---|---|---|---|---|
| Lab 1 | 324 | 284 | 288 | 263 |
| Lab 2 | 324 | 281 | 284 | 263 |

Results where comparative data were missing (sampling 2 - 3 results, sampling 3 - 4 results) were not taken into account in the reproducibility calculations.

**Table 14. Reproducibility - 80-120% (p < 0.05) determined on the basis of data obtained in an inter-laboratory study (n.d. - not available).**

| **Drug** | **Venous blood** | | | **Capillary blood** (Mitra^{®}) | | |
|---|---|---|---|---|---|---|
| | Reproducibility [%] | CV (%) | p < 0.05 | Reproducibility [%] | CV (%) | p < 0.05 |
| Amiodarone | 108.00 | 2.27 | ✔ | 106.00 | 11.60 | ✔ |
| Propafenone | 99.90 | 6.43 | ✔ | 100.89 | 11.48 | ✔ |
| Sotalol | 99.28 | 4.06 | ✔ | 99.55 | 10.12 | ✔ |
| Digoxin | 103.98 | 4.79 | ✔ | 103.81 | 19.89 | ✔ |
| Ramipril | 87.92 | 19.68 | ✔ | 103.49 | 16.39 | ✔ |
| Eplerenone | 115.66 | 7.80 | ✔ | 108.20 | 18.11 | ✔ |
| Desetyl-Amiodarone | 100.97 | 6.73 | ✔ | 111.93 | 11.79 | ✔ |
| Hydroxy-Propafenone | 98.07 | 14.28 | ✔ | 94.56 | 22.09 | ✔ |
| Spironolactone | 91.65 | 12.29 | ✔ | 96.45 | 19.13 | ✔ |
| Metoprolol | 136.42 | 5.96 | ✔ | 132.84 | 20.05 | ✔ |
| Perindopril | 112.62 | 17.46 | ✔ | 109.23 | 15.30 | ✔ |
| Carvedilol | 98.17 | 5.88 | ✔ | 100.56 | 9.66 | ✔ |
| Bisoprolol | 119.52 | 7.80 | ✔ | 115.39 | 17.97 | ✔ |
| Nebivolol | 103.78 | 2.89 | ✔ | 101.60 | 8.57 | ✔ |

On the basis of the results of quantitative analyses obtained from samples taken from patients during outpatient clinic visits, the correlation coefficients of venous blood vs. capillary blood (V/C) and the precision of the test were determined. The obtained ratios of individual substances in venous and capillary blood were averaged and divided by each other to obtain the correlation coefficient. On the basis of all replicates for each analyte, the precision of the test and the statistical significance of the correlation were determined. The results are shown in Table 15.

**Table 15. Correlations - venous blood vs capillary blood (V/C). Test precision (±10%, p < 0.05).**

| **Drug** | **V/C ratio** | **Precision [%]** | **p** < **0.05** |
|---|---|---|---|
| Amiodarone | 2.10 | 2.95 | ✔ |
| Propafenone | 1.82 | 9.88 | ✔ |
| Sotalol | 0.94 | 6.05 | ✔ |
| Digoxin | 0.69 | 1.22 | ✔ |
| Ramipril | 1.15 | 7.90 | ✔ |
| Eplerenone | 1.78 | 5.12 | ✔ |
| Desethyl-Am iodarone | 1.28 | 12.43 | ✔ |
| Hydroxy-Propafenone | 1.00 | 7.33 | ✔ |
| Spironolactone | 1.76 | 5.19 | ✔ |
| Metoprolol | 0.87 | 9.79 | ✔ |
| Perindopril | 1.86 | 4.91 | ✔ |
| Carvedilol | 1.86 | 4.89 | ✔ |
| Bisoprolol | 1.06 | 4.35 | ✔ |
| Nebivolol | 1.04 | 1.71 | ✔ |

The reproducible (±10%) and significantly statistical (p < 0.05) correlation coefficients determined for the tested drugs confirm the effectiveness of the diagnostic method based on the analysis of dry capillary blood collected on sorbent. These coefficients can also be used to estimate the concentration of drugs in venous blood based on the analysis of their concentration in capillary blood.

## Claims

1. A method for monitoring a panel of antiarrhythmic drugs in a blood of a subject, the method comprising:
- obtaining a blood sample from the subject; and
- determining in the blood sample, using liquid chromatography tandem mass spectrometry (LC-MS/MS), a level of at least one antiarrhythmic drug or antiarrhythmic drug metabolite selected from a group consisting of: Amiodarone, Propafenone, Sotalol, Digoxin, Ramipril, Eplerenone, Desetyl-Amiodarone, Hydroxy-Propafenone, Spironolactone, Metoprolol, Perindopril, Carvedilol, Bisoprolol and Nebivolol.

2. The method of claim 1 wherein the blood sample is a venous blood sample.

3. The method of claim 1 wherein the blood sample is a capillary blood sample.

4. The method of claim 3 wherein the capillary blood sample is collected on a sorbent.

5. The method of claim 3 or 4, further comprising converting the antiarrhythmic drug level in capillary blood to the antiarrhythmic drug level in venous blood by multiplying the antiarrhythmic drug level in capillary blood by a predetermined V/C ratio.

6. A computer-implemented system for monitoring a panel of antiarrhythmic drugs in a blood of a subject, the system comprising:
- a liquid chromatography tandem mass spectrometry (LC-MS/MS) apparatus (20) configured to receive a blood sample (10) and to perform LC-MS/MS analysis to output LS-MS/MS measurement data; and
- an analytical module (30) configured to receive the LS-MS/MS measurement data, the analytical module (30) comprising at least one nontransitory processor-readable storage medium (31) that stores at least one of processor-executable instructions (33) and data (32) and at least one processor (34) communicably coupled to the storage medium (31) and configured to, by executing the instructions (33) and based on the data (32), determine in the blood sample a level of at least one antiarrhythmic drug or antiarrhythmic drug metabolite selected from a group consisting of: Amiodarone, Propafenone, Sotalol, Digoxin, Ramipril, Eplerenone, Desetyl-Amiodarone, Hydroxy-Propafenone, Spironolactone, Metoprolol, Perindopril, Carvedilol, Bisoprolol and Nebivolol.
